# EUROPÄISCHE PATENTSCHRIFT

(11) **EP 3 615 680 B1**
(45) Veröffentlichungstag und Bekanntmachung des Hinweises auf die Patenterteilung: **30.08.2023**
(21) Anmeldenummer: 18719074.9
(22) Anmeldetag: 06.04.2018
(51) Int. Cl.: C12Q 1/24, C12N 5/00, G01N 21/65, C12M 1/26, C12M 1/42

(54) **VERFAHREN UND VORRICHTUNG ZUR BEREITSTELLUNG EINER EINE GEWÜNSCHTE ZIELPROTEIN-EXPRESSION AUFWEISENDEN ZELLLINIE**
METHOD AND SYSTEM FOR PREPARATION OF A TARGET PROTEIN EXPRESSING CELL LINE
PROCÉDÉ ET DISPOSITIF PERMETTANT D'OBTENIR UNE LIGNÉE CELLULAIRE AYANT UNE CERTAINE EXPRESSION D'UNE PROTÉINE CIBLE SOUHAITÉE

(30) Priorität: 28.04.2017 DE 102017207262
(43) Veröffentlichungstag der Anmeldung: 04.03.2020
(73) Patentinhaber: Fraunhofer-Gesellschaft zur Förderung der angewandten Forschung e.V., 80686 München (DE)
(72) Erfinder: GILLNER, Arnold, 52159 Roetgen (DE); NOTTRODT, Nadine, 52072 Aachen (DE); WEHNER, Martin, 52134 Herzogenrath (DE); RIESTER, Dominik, 81245 München (DE); BURGER-KENTISCHER, Anke, 70569 Stuttgart (DE); BACH, Monika, 70771 Leinfelden-Echterdingen (DE); BRAUCHLE, Eva, 72070 Tübingen (DE); SCHENKE-LAYLAND, Katja, 72070 Tübingen (DE); GREINER, Benjamin, 53639 Königswinter (DE); PIPPOW, Andreas, 50968 Köln (DE); NGUYEN, Phuong-Ha, 53227 Bonn (DE); HOFER-SCHMITZ, Katharina, 8020 Graz (AT)
(74) Vertreter: Schrell, Andreas
(86) Internationale Anmeldenummer: PCT/EP2018/058883
(87) Internationale Veröffentlichungsnummer: WO 2018/197183

(56) Entgegenhaltungen:
- WO-A2-2006/130728
- YIZHI SONG ET AL: "Single-cell genomics based on Raman sorting reveals novel carotenoid-containing bacteria in the Red Sea", MICROBIAL BIOTECHNOLOGY, Bd. 10, Nr. 1, 17. Oktober 2016 (2016-10-17), Seiten 125-137, XP055484411, GB ISSN: 1751-7915, DOI: 10.1111/1751-7915.12420
- SONG YIZHI ET AL: "Raman activated cell sorting", CURRENT OPINION IN CHEMICAL BIOLOGY, CURRENT BIOLOGY LTD, LONDON, GB, Bd. 33, 18. April 2016 (2016-04-18), Seiten 1-8, XP029723104, ISSN: 1367-5931, DOI: 10.1016/J.CBPA.2016.04.002
- YUN WANG ET AL: "Raman Activated Cell Ejection for Isolation of Single Cells", ANALYTICAL CHEMISTRY, Bd. 85, Nr. 22, 19. November 2013 (2013-11-19), Seiten 10697-10701, XP055370214, US ISSN: 0003-2700, DOI: 10.1021/ac403107p
- PAHLOW SUSANNE ET AL: "Isolation and identification of bacteria by means of Raman spectroscopy", ADVANCED DRUG DELIVERY REVIEWS, ELSEVIER, AMSTERDAM, NL, Bd. 89, 17. April 2015 (2015-04-17), Seiten 105-120, XP029257237, ISSN: 0169-409X, DOI: 10.1016/J.ADDR.2015.04.006
- Marinella G Sandros: "Raman Spectroscopy and microscopy enable life science discoveries", Photonics, 1. März 2015 (2015-03-01), Seiten 1-4, XP055507818, Gefunden im Internet: URL:https://pdfs.semanticscholar.org/dc79/ a63c7f1d566b7f909b5ea4f8bd8147a0e8eb.pdf [gefunden am 2018-09-18]
- MENGQIU LI ET AL: "Single cell Raman spectroscopy for cell sorting and imaging", CURRENT OPINION IN BIOTECHNOLOGY., Bd. 23, Nr. 1, 1. Februar 2012 (2012-02-01), Seiten 56-63, XP055508071, GB ISSN: 0958-1669, DOI: 10.1016/j.copbio.2011.11.019
- Changhan Xie: "Raman sorting and identification of single living micro-organisms with optical tweezers", Optics Letters, 15. Juli 2005 (2005-07-15), Seiten 1800-1802, XP055507814, Gefunden im Internet: URL:https://www.osapublishing.org/DirectPD FAccess/3E3C309C-015E-713E-C5E1198843274AC 0_84601/ol-30-14-1800.pdf?da=1&id=84601&se q=0&mobile=no [gefunden am 2018-09-18]
- WEI E. HUANG ET AL: "Raman tweezers sorting of single microbial cells", ENVIRONMENTAL MICROBIOLOGY REPORTS, Bd. 1, Nr. 1, 1. Februar 2009 (2009-02-01) , Seiten 44-49, XP055507794, GB ISSN: 1758-2229, DOI: 10.1111/j.1758-2229.2008.00002.x
- MENGQIU LIDAN G BOARDMANANDREW WARDWEI E HUANG ED - IAN T PAULSEN ET AL: "Single-Cell Raman Sorting", [Online] 1. Januar 2014 (2014-01-01), ENVIRONMENTAL MICROBIOLOGY: METHODS AND PROTOCOLS; [METHODS IN MOLECULAR BIOLOGY; ISSN 1940-6029; VOL. 1096], SPRINGER, US, PAGE(S) 147 - 153, XP009508150, ISBN: 978-1-62703-711-2 Gefunden im Internet: URL:https://link.springer.com/protocol/10. 1007%2F978-1-62703-712-9_12> [gefunden am 2014-01-15] das ganze Dokument
- LAU ADRIAN Y ET AL: "An integrated optofluidic platform for Raman-activated cell sorting", LAB ON A CHIP, ROYAL SOCIETY OF CHEMISTRY, Bd. 8, 28. Mai 2008 (2008-05-28), Seiten 1116-1120, XP002519719, ISSN: 1473-0197, DOI: 10.1039/B803598A
- Rainer Uhl: "Förderinitiative "Ultrasensitiver Nachweis und Manupulation von Zellen bzw. Geweben und ihren molekularen Bestandteilen". Identifizierung und Charakterisierung von Blut zirkulierenden Tumomrzellen mittels Raman-Spektroskopie, Mikrofluidik und Mikroarrays (Raman CTC)", , 30. November 2016 (2016-11-30), Seiten 1-2, XP055507648, Gefunden im Internet: URL:https://www.photonikforschung.de/media /lebenswissenschaften/pdf/1-bf-Psb_RAMAN-C TC_korr2016-11_bf_C1.pdf [gefunden am 2018-09-18]
- Batirtze Prats Mateu ET AL: "Label-free live cell imaging by confocal Raman microscopy identifies CHO host and producer cell lines", Biotechnology Journal, 21. Juli 2016 (2016-07-21), Seiten 1-8, XP055484699, Gefunden im Internet: URL:https://onlinelibrary.wiley.com/doi/pd f/10.1002/biot.201600037 [gefunden am 2018-06-15]
- Eva Brauchle ET AL: "Raman microspectroscopy for the development and screening of recombinant cell lines", Biotechnology Journal, 23. September 2016 (2016-09-23), Seiten 1-3, XP055484349, Gefunden im Internet: URL:https://onlinelibrary.wiley.com/doi/pd f/10.1002/biot.201600412 [gefunden am 2018-06-14]
- EVA BRAUCHLE ET AL: "Cell death stages in single apoptotic and necrotic cells monitored by Raman microspectroscopy", SCIENTIFIC REPORTS, Bd. 4, Nr. 1, 15. April 2014 (2014-04-15), Seiten 1-9, XP055484384, DOI: 10.1038/srep04698
- BROWNE ET AL: "Selection methods for high-producing mammalian cell lines", TRENDS IN BIOTECHNOLOGY, ELSEVIER PUBLICATIONS, CAMBRIDGE, GB, Bd. 25, Nr. 9, 23. August 2007 (2007-08-23), Seiten 425-432, XP022210243, ISSN: 0167-7799, DOI: 10.1016/J.TIBTECH.2007.07.002
- TINGFENG LAI ET AL: "Advances in Mammalian Cell Line Development Technologies for Recombinant Protein Production", PHARMACEUTICALS, Bd. 6, Nr. 5, 26. April 2013 (2013-04-26), Seiten 579-603, XP055290466, DOI: 10.3390/ph6050579
- ANGELA WALTER ET AL: "Towards a fast, high specific and reliable discrimination of bacteria on strain level by means of SERS in a microfluidic device", LAB ON A CHIP, Bd. 11, Nr. 6, 31. Januar 2011 (2011-01-31), Seite 1013, XP055260408, ISSN: 1473-0197, DOI: 10.1039/c0lc00536c
- KARL OTTO GREULICH: "Manipulation of cells with laser microbeam scissors and optical tweezers: a review", REPORTS ON PROGRESS IN PHYSICS, INSTITUTE OF PHYSICS PUBLISHING, BRISTOL, GB, Bd. 80, Nr. 2, 23. Dezember 2016 (2016-12-23), Seite 26601, XP020312626, ISSN: 0034-4885, DOI: 10.1088/1361-6633/80/2/026601 [gefunden am 2016-12-23]
- F. Schaal ET AL: "Marker-free cell discrimination by holographic optical tweezers", Journal of the European Optical Society - Rapid Publications, 3. Juni 2009 (2009-06-03), Seiten 09028-1, XP055006233, DOI: 10.2971/jeos.2009.09028 Gefunden im Internet: URL:http://www.jeos.org/index.php/jeos_rp/ article/viewFile/09028/426 [gefunden am 2011-09-05]
- ALESSIA PALLAORO ET AL: "Rapid Identification by Surface-Enhanced Raman Spectroscopy of Cancer Cells at Low Concentrations Flowing in a Microfluidic Channel", ACS NANO, AMERICAN CHEMICAL SOCIETY, US , Bd. 9, Nr. 4 28. April 2015 (2015-04-28), Seiten 4328-4336, XP009508125, ISSN: 1936-0851, DOI: 10.1021/ACSNANO.5B00750 Gefunden im Internet: URL:https://pubs.acs.org/doi/pdfplus/10.10 21/acsnano.5b00750 [gefunden am 2015-03-17]

## Beschreibung

Die Erfindung betrifft ein Verfahren zur Bereitstellung einer eine gewünschte Zielprotein-Expression aufweisenden Zelllinie sowie eine Vorrichtung zur Selektion von eine gewünschte Zielprotein-Expression aufweisenden Zelllinien.

Die Herstellung von effizienten Produktionszelllinien (High-Producer Zellen) ist ein essentieller Schritt bei der biotechnologischen Herstellung pharmazeutischer Wirkstoffe. Heute erfolgt die Auswahl von Zellen, die eine besonders hohe Expressionsrate eines gewünschten Zielproteins aufweisen, über aufwändige, insbesondere zeit- und kostenintensive, meist auf immunologischen Methoden beruhenden Screeningverfahren zur Bestimmung der exprimierten Menge des Zielproteins. Es besteht daher ein großer Bedarf an schnellen Automatisierungsverfahren zur Selektion bestimmter Zellen in Abhängigkeit der Expression der gewünschten Zielproteine, um kosten- und zeitgünstigere Herstellungsverfahren geeigneter Produktionszelllinien zu etablieren.

Im Stand der Technik werden zur Selektion geeigneter Produktionszelllinien transfizierte Zellen mittels Fluoreszenzverfahren hinsichtlich der Expressionsleistung für das Zielprotein analysiert. Hierzu werden üblicherweise Fluoreszenzmarker eingesetzt, die das Zielprotein im Überstand der Zellkulturen markieren und mittels gängiger Fluoreszenzanalytik-Verfahren detektiert werden können. Dies erfordert jedoch einerseits die Kenntnis der Struktur des Zielproteins und andererseits das Vorhandensein eines geeigneten Markersystems. In gängigen Verfahren werden die Klone beispielsweise mit fluoreszenzmarkierten Antikörpern, die an das Zielprotein binden, markiert und die segregierte Menge an Zielprotein mittels Immunofluoreszenzverfahren bestimmt. Nach der Analyse der mittels Fluoreszenzverfahren ausgewählten Zellen werden diese mittels manueller Techniken oder mittels sogenannter Zellpicker aus der Zellkolonie isoliert und zur weiteren Expansion als Klon vereinzelt. Zur automatischen Selektion von High-Producer Zellen werden kommerziell Geräte angeboten, mit denen kleine Zellkolonien aus Kulturschalen entnommen und transferiert werden können. Dazu werden zum Beispiel Metallnadeln eingesetzt, die auf die Zellkolonie aufgedrückt werden, sodass die daran anhaftenden Zellen gezielt entnommen werden können. Alternativ werden Mikrodispenser angeboten, die über eine Glaskapillare die Zellen mit Flüssigkeit aufnehmen und an anderer Stelle wieder absetzen können. Diese Systeme sind üblicherweise auch mit Einrichtungen zur Fluoreszenzanalyse und Bildverarbeitung ausgerüstet, um die einzelnen Zellklone zu identifizieren. Die Positionierung der Nadeln und Kanülen zur Entnahme einzelner Klone kann dabei über die Bildverarbeitungssoftware automatisiert gesteuert werden. Ergänzend können Hellfeldmikroskopie oder Phasenkontrastverfahren zur Analyse der Zellmorphologie eingesetzt werden. Jedoch ist es mit den bisher im Stand der Technik bekannten Systemen nicht möglich, bereits auf ihre Proteinexpression hin analysierte Zellen gezielt aus einer Kultur einzeln zu entnehmen.

Nachteilig bei den bisherigen Methoden ist somit insbesondere der Umstand, dass die Zielzellen zur Feststellung der Expressionsrate für das Zielprotein markiert werden müssen und die bisher im Stand der Technik beschriebenen Selektionstechniken nicht die nötige Analysetechnik aufweisen, um auf ihre Proteinexpression hin analysierte Einzelzellen zu isolieren. Zudem kann die Zellselektion bisher erst nach ausreichender Expression des Zielproteins im Überstand der Zelle erfolgen. In herkömmlichen Verfahren dauert die Kultivierung der Klone mehrere Wochen bis Monate.

Der vorliegenden Erfindung liegt daher das technische Problem zugrunde, ein Verfahren zur Bereitstellung einer eine gewünschte Zielprotein-Expression aufweisenden Zelllinie bereitzustellen, insbesondere ein solches Verfahren, das die vorgenannten Nachteile überwindet. Der Erfindung liegt auch das technische Problem zugrunde, eine Vorrichtung zur Selektion von eine gewünschte Zielprotein-Expression aufweisenden Zelllinien bereitzustellen.

Die vorliegende Erfindung löst das ihr zugrundeliegende Problem durch die Lehre der unabhängigen Ansprüche.

Das erfindungsgemäße Verfahren zur Bereitstellung einer eine gewünschte Zielprotein-Expression aufweisenden Zelllinie umfasst dabei die folgenden Schritte:
a) Bereitstellen von Zellen, die jeweils mindestens eine Nukleotidsequenz aufweisen, die mindestens ein Zielprotein kodiert,
b) Charakterisieren der Proteinexpression der in Schritt a) bereitgestellten Zellen auf Einzelzellebene mittels Raman-Spektroskopie,
c) Selektieren mindestens einer Zelle, die eine gewünschte Zielprotein-Expression aufweist,
d) Transferieren der mindestens einen in Schritt c) selektierten Zelle in ein Expansionsmedium mittels Laser Induced Forward Transfer (LIFT), wobei der verwendete Transferträger keine Absorberschicht aufweist, wobei die Transfermaterial-Schicht als Absorberschicht dient und wobei für den Laser Induced Forward Transfer (LIFT) eine Laserstrahlquelle mit einer Wellenlänge von > 3 µm verwendet wird,
e) Kultivieren der mindestens einen selektierten Zelle in einem Expansionsmedium,
f) Charakterisieren der quantitativen Proteinexpression der in Schritt e) kultivierten mindestens einen Zelle auf Einzelzellebene mittels zeitaufgelöster Raman-Spektroskopie, und
g) Selektieren einer die gewünschte Zielprotein-Expression aufweisenden Zelllinie.

In einer besonders bevorzugten Ausführungsform werden die Verfahrensschritte a) bis g) in der angegebenen Reihenfolge durchgeführt. In besonders bevorzugter Ausführungsform besteht das vorliegende Verfahren aus den vorliegenden Verfahrensschritten, das heißt zwischen den einzelnen Verfahrensschritten finden keine weitere Verfahrensschritte statt, vorzugsweise sind weder vor noch nach dem Durchführen der ohne Zwischenschritte durchgeführten Verfahrensschritte a) bis g) weitere Verfahrensschritte zur Bereitstellung der die gewünschte Zielprotein-Expression aufweisenden Zelllinie vorgesehen.

Das erfindungsgemäße Verfahren stellt demgemäß vorteilhafterweise eine Kombination aus, insbesondere markerfreier, Charakterisierung der Proteinexpression von Zellen auf Einzelzellebene gemäß Verfahrensschritt b) und einer, insbesondere laserbasierten, Einzelzellselektion gemäß Verfahrensschritt c) bereit, bei der gemäß Verfahrensschritt b) die Expression eines Zielproteins bereits innerhalb einer einzelnen Zelle nachgewiesen werden kann und wobei die so analysierte Zelle direkt in einem einzigen Verfahrensschritt für die weitere Herstellung einer eine gewünschte Zielprotein-Expression aufweisenden Zelllinie selektioniert und vereinzelt werden kann.

Die Erfindung ermöglicht es in effizienter und einfacher Weise, eine eine gewünschte Zielprotein-Expression aufweisende Zelllinie mit hoher Präzision und Verlässlichkeit bereitzustellen, insbesondere in kurzer Zeit.

Unter einer "gewünschten Zielprotein-Expression" wird im Zusammenhang mit der vorliegenden Erfindung verstanden, dass eine für einen bestimmten Einsatzzweck der Zelllinie angestrebte Expression eines Zielproteins, zum Beispiel eine bestimmte angestrebte, z.B. hohe oder niedrige, Expressionsmenge oder eine bestimmte angestrebte, z.B. hohe oder niedrige, Expressionsrate des Zielproteins, von einer bereitgestellten Zelllinie, insbesondere stabil und über einen längeren Zeitraum, realisiert wird.

Im Zusammenhang mit der vorliegenden Erfindung wird mit dem Begriff "Zielprotein" das Protein bezeichnet, dessen Expression in einer Zelle von Interesse ist. Dabei kann es sich erfindungsgemäß sowohl um ein in den bereitgestellten Zellen natürlich vorkommendes Protein handeln als auch um ein Protein, dessen Präsenz und/oder Expression in den bereitgestellten Zellen artifiziell gezielt herbeigeführt wurde.

Im Zusammenhang mit der vorliegenden Erfindung wird unter einer "Nukleotidsequenz" die Abfolge der Nukleotide einer Nukleinsäure verstanden. Bevorzugt ist die Nukleotidsequenz eine DNA-Sequenz oder eine RNA-Sequenz, insbesondere eine DNA-Sequenz.

Erfindungsgemäß wird unter dem Begriff "Zellen" verstanden, dass mindestens zwei Zellen, vorzugsweise mehrere, insbesondere mindestens 10³, 10⁴, 10⁵, 10⁶, 10⁷, 10⁸ oder mehr Zellen vorliegen. Die in Schritt a) bereitgestellten Zellen können einen Anteil einer eine noch größere Anzahl an Zellen aufweisenden Zellpopulation darstellen. So ist es denkbar, dass in Schritt a) eine Zellpopulation vorliegt, von der nur eine bestimmte Anzahl an Zellen jeweils mindestens eine Nukleotidsequenz aufweist, die mindestens ein Zielprotein kodiert und die restlichen Zellen eine solche Nukleotidsequenz nicht aufweisen.

Im Zusammenhang mit der vorliegenden Erfindung wird unter "unüberwacht" verstanden, dass in Schritt b) erhaltene Raman-Spektren ungelabelt sind bezüglich der Proteinexpression der in Schritt a) bereitgestellten Zellen, das heißt die zu erwartenden Spektren der Expressionsmuster nicht bekannt sind. Unter "teilüberwacht" wird im Zusammenhang mit der vorliegenden Erfindung verstanden, dass in Schritt b) erhaltene Raman-Spektren teilweise gelabelt sind bezüglich der Proteinexpression der in Schritt a) bereitgestellten Zellen, das heißt die zu erwartenden Spektren der Expressionsmuster teilweise bekannt sind.

Die Erfindung sieht vor, in Verfahrensschritt a) Zellen bereitzustellen, wobei die bereitgestellten Zellen jeweils mindestens eine Nukleotidsequenz aufweisen, die mindestens ein Zielprotein kodiert.

In einer bevorzugten Ausführungsform sind die in Schritt a) bereitgestellten Zellen tierische Zellen, bevorzugt Säugerzellen, bevorzugt Nagetierzellen, bevorzugt Mauszellen, bevorzugt Hamsterzellen, bevorzugt Zellen des chinesischen Zwerghamsters (Cricetulus griseus), insbesondere Ovarienzellen des chinesischen Zwerghamsters (CHO-Zellen), bevorzugt NS0-Zellen, bevorzugt PERC6-Zellen, bevorzugt HeLa-Zellen, bevorzugt HEK293-Zellen, bevorzugt Nierenzellen des chinesischen Zwerghamsters (BHK-Zellen).

Bevorzugt sind die in Schritt a) bereitgestellten Zellen Säugerzellen. Bevorzugt sind die in Schritt a) bereitgestellten Zellen keine Säugerzellen.

Bevorzugt sind die in Schritt a) bereitgestellten Zellen Insektenzellen. Bevorzugt sind die in Schritt a) bereitgestellten Zellen keine Insektenzellen.

Bevorzugt sind die in Schritt a) bereitgestellten Zellen Mikroorganismen. Bevorzugt sind die in Schritt a) bereitgestellten Zellen keine Mikroorganismen.

Bevorzugt sind die in Schritt a) bereitgestellten Zellen bakterielle Zellen. Bevorzugt sind die in Schritt a) bereitgestellten Zellen keine bakteriellen Zellen.

In einer bevorzugten Ausführungsform ist die mindestens eine, mindestens ein Zielprotein kodierende Nukleotidsequenz auf einem Plasmid angeordnet.

In einer bevorzugten Ausführungsform liegt die mindestens eine, mindestens ein Zielprotein kodierende Nukleotidsequenz stabil oder transient in das Genom der Zellen integriert vor. Bevorzugt liegt die mindestens eine, mindestens ein Zielprotein kodierende Nukleotidsequenz stabil in das Genom der Zellen integriert vor. Bevorzugt liegt die mindestens eine, mindestens ein Zielprotein kodierende Nukleotidsequenz transient in das Genom der Zellen integriert vor.

Die mindestens eine, mindestens ein Zielprotein kodierende Nukleotidsequenz kodiert bevorzugt genau ein Zielprotein. Bevorzugt kodiert die mindestens eine, mindestens ein Zielprotein kodierende Nukleotidsequenz genau zwei, bevorzugt genau drei, bevorzugt genau vier, bevorzugt genau fünf Zielproteine. Bevorzugt kodiert die mindestens eine mindestens ein Zielprotein kodierende Nukleotidsequenz mindestens zwei Zielproteine, bevorzugt mindestens drei, mindestens vier oder mindestens fünf Zielproteine. Bevorzugt kodiert die mindestens eine mindestens ein Zielprotein kodierende Nukleotidsequenz eine beliebige Anzahl an Zielproteinen.

In einer bevorzugten Ausführungsform findet vor Schritt a) eine Transfektion von Zellen mit mindestens einer Nukleotidsequenz, die mindestens ein Zielprotein kodiert, statt.

Bevorzugt werden zur Transfektion der Zellen dem Fachmann bekannte Verfahren angewendet. Bevorzugt ist das Verfahren zur Transfektion von Zellen ausgewählt aus Kalziumphosphat-Präzipitation, Lipofektion, Transfektion mittels kationischer Polymere, Mikroinjektion, Elektroporation, Particle Gun, Magnetofektion, Sonoporation, Transferinfektion und antikörpervermittelte Transfektion.

In einer bevorzugten Ausführungsform erfolgt vor Schritt a) eine Selektion transfizierter Zellen. Bevorzugt erfolgt vor Schritt a) keine Selektion transfizierter Zellen.

Bevorzugt weisen alle in Schritt a) bereitgestellten Zellen jeweils mindestens eine Nukleotidsequenz auf, die mindestens ein Zielprotein kodiert. Bevorzugt weist nur ein Teil der in Schritt a) bereitgestellten Zellen jeweils mindestens eine Nukleotidsequenz auf, die mindestens ein Zielprotein kodiert.

Erfindungsgemäß erfolgt in Verfahrensschritt b) ein Charakterisieren der Proteinexpression der in Schritt a) bereitgestellten Zellen auf Einzelzellebene mittels Raman-Spektroskopie. Ein Charakterisieren der Proteinexpression von Zellen auf Einzelzellebene bedeutet im Zusammenhang mit der vorliegenden Erfindung, dass eine Analyse der Expression, zum Beispiel der Expressionsrate oder der Expressionsmenge, eines bestimmten Proteins individuell für eine bestimmte einzelne Zelle durchgeführt wird.

Das für die erfindungsgemäß vorgesehene Raman-Spektroskopie eingesetzte optische System umfasst in bevorzugter Ausführungsform ein Raman-Spektrometer und mindestens ein Mikroskop. Im Zusammenhang mit der vorliegenden Erfindung umfasst ein Raman-Spektrometer mindestens eine Lichtquelle, insbesondere mindestens eine Anregungslichtquelle, insbesondere mindestens einen Laser, und mindestens einen Detektor. Zudem können mikrofluidische Systeme mit einer direkten Lichtkopplung über unmittelbar an die Zellen herangebrachten Lichtleitfasern realisiert werden, die eine hohe numerische Apertur beziehungsweise Sammeleffizienz der Signalerfassung ohne ein Hinzufügen weiterer optischer Elemente erlauben.

Die zur Analyse der Proteinexpression eingesetzte Raman-Spektroskopie ermöglicht es vorteilhafterweise, Molekülstrukturen mit hoher Auflösung auf Einzelzellebene zu ermitteln. Dabei wird die Untersuchung von Zellen auf Einzelzellebene insbesondere durch die Kopplung des Raman-Spektrometers an ein Mikroskop oder Lichtsammler mit hoher numerischer Apertur realisiert. Bei der Raman-Spektroskopie werden Molekülschwingungen detektiert, wodurch es möglich ist, ohne die Notwendigkeit der Verwendung eines Markers Feststoffe, bestimmte Flüssigkeiten und/oder Gase sowie Biomoleküle zu identifizieren. Spezifische Schwingungsmuster der in den Zellen oder im Überstand enthaltenen Proteine können mit einem Laser im sichtbaren oder nahinfraroten Bereich angeregt werden, ohne dadurch die Zellviabilität negativ zu beeinflussen. Das rückgestreute Licht weist dabei eine durch Energieverlust geringfügig veränderte Wellenlänge auf. Auf diese Weise können mit Hilfe der detektierten Spektren Unterschiede und spezifische Merkmale in der Proteinzusammensetzung einer einzelnen Zelle ermittelt werden. Die Analyse der Zellen erfolgt dabei bevorzugt innerhalb eines optischen Systems, bei dem die Zellen in einem Mikroskopstrahlengang bestrahlt und das rückgestreute und frequenzverschobene Licht analysiert wird.

In einer besonders bevorzugten Ausführungsform der vorliegenden Erfindung ist die Raman-Spektroskopie eine Surface-Enhanced-Raman-Spektroskopie (SERS).

In einer bevorzugten Ausführungsform erfolgt das Charakterisieren der Proteinexpression der in Schritt a) bereitgestellten Zellen auf Einzelzellebene in Schritt b) dementsprechend mittels Surface-Enhanced Raman-Spektroskopie (SERS), bevorzugt mittels quantitativer Surface-Enhanced Raman-Spektroskopie.

Surface-Enhanced Raman-Spektroskopie (SERS) liefert im Vergleich zum klassischen Raman-Effekt wesentlich intensivere Signale und kommt insbesondere dann zum Einsatz, wenn die Intensität der bei der Raman-Spektroskopie detektierten Signale, beispielsweise aufgrund der zu geringen Materialmenge, sehr gering ist. SERS beruht auf der lokalen Feldüberhöhung an elektrisch leitfähigen Partikeln oder Nanostrukturen und ermöglicht es, die Intensität des erhaltenen Raman-Signals im Vergleich zur klassischen Raman-Spektroskopie um einen Faktor von 10³ bis 10⁶ zu erhöhen. So können beispielsweise kolloidale Goldschichten als Trägersubstrate verwendet werden, was insbesondere bei sekretierten Proteinen, wie Antikörpern zum Einsatz kommen kann. Eine weitere Möglichkeit besteht darin, Kupfer-, Platin-, Palladium-, Silber-, oder Goldnanopartikel in Zellen einzuschleusen, um so die intrazellulären Signale zu verstärken.

Die vorliegende Erfindung ermöglicht es somit vorteilhafterweise in Schritt b) die Proteinexpression einzelner Zellen mittels Raman-Spektroskopie, bevorzugt mittels Surface-Enhanced Raman-Spektroskopie (SERS), zu charakterisieren.

In einer bevorzugten Ausführungsform werden im erfindungsgemäßen Verfahren keine Marker, insbesondere keine Fluoreszenzmarker, eingesetzt. Bevorzugt ist das erfindungsgemäße Verfahren markerfrei.

In einer weiteren bevorzugten Ausführungsform der vorliegenden Erfindung erfolgt nach Schritt b) eine Klassifizierung der in Schritt b) charakterisierten Zellen mittels Hauptkomponentenanalyse (PCA).

In einer weiteren bevorzugten Ausführungsform der vorliegenden Erfindung erfolgt nach Schritt b) eine Klassifizierung der in Schritt b) charakterisierten Zellen mittels unüberwachten oder teilüberwachten Ensemble Machine Learning Methoden und/oder Analyse mittels neuronaler Netzwerke.

Bevorzugt werden in Schritt b) die Raman-Spektren, die verschiedene spektrale Signaturen (welche mit verschiedenen Zielprotein-Expressionen korrelieren) mit Ensemble Machine Learning Methoden getrennt. Hierzu werden die verrauschten Spektren mit geeigneten Verfahren (beispielsweise mit Baseline-Correction, Wavelets oder Hauptkomponentenanalyse (PCA)) vorverarbeitet. Dann werden zur Trennung der Spektren insbesondere unüberwachte und teilüberwachte Ensemble Machine Learning Methoden eingesetzt. Der Vorteil dieses Verfahrens ist, dass in der untersuchten Menge von spektralen Signaturen solche Signaturen gefunden werden, die a priori nicht bekannt sind und der gesuchten Zielprotein-Expression entsprechen.

Erfindungsgemäß wird in Schritt c) mindestens eine Zelle selektiert, die gemäß der in Schritt b) durchgeführten Charakterisierung eine gewünschte Zielprotein-Expression aufweist. Bevorzugt erfolgt die Selektion der mindestens einen die gewünschte Zielprotein-Expression aufweisenden Zelle automatisiert.

Erfindungsgemäß wird die mindestens eine in Schritt c) selektierte Zelle in Schritt d) in ein Expansionsmedium transferiert.

Die vorliegende Erfindung sieht in bevorzugter Ausführung und vorteilhafterweise in Schritt d) vor, einzelne in Schritt c) selektierte Zellen in ein jeweils den einzelnen Zellen jeweils zugeordnetes Expansionsmedium zu transferieren.

Gemäß der vorliegenden Erfindung erfolgt das Transferieren der mindestens einen selektierten Zelle in ein Expansionsmedium gemäß Schritt d) mittels einer laserbasierten Zelltransfertechnik.

Erfindungsgemäß handelt es sich bei der laserbasierten Zelltransfertechnik um ein Laser Induced Forward Transfer (LIFT)-Verfahren, das für biologische Materialien auch Laser Assisted Bioprinting genannt wird.

Beim Laser Induced Forward Transfer (LIFT)-Verfahren wird eine geringe Materialmenge mittels eines gezielten Laserpulses von einem Transferträger auf ein Zielsubstrat übertragen. Der Transferträger besteht in der Regel aus drei Schichten, einer für das Laserlicht transparenten Trägerschicht, einer Absorber- und einer Transfermaterial-Schicht. Im Stand der Technik sind jedoch auch Varianten bekannt, in denen anstelle von Absorberschichten absorbierende Trägerschichten eingesetzt werden. Durch einen Laserimpuls wird die Absorberschicht/absorbierende Trägerschicht kurzzeitig erwärmt und eine Dehnungswelle oder eine Dampfblase erzeugt, die den Transport einer kleinen Materialmenge ähnlich einem Düsendruckkopf auslöst. Der entstandene Tropfen oder Partikel kann auf einem Empfängerträger, z.B. einem Objektträger oder einer Mikrotiterplatte, in einer Entfernung von etwa 0,1 bis 1 mm aufgefangen werden. Dabei können die zu übertragenden Materialien als flüssige, hochviskose oder feste Stoffe vorliegen. Die zu übertragenden Materialien können auch als Kombination verschiedener Stoffe, wie beispielsweise Flüssigkeiten und Zellen vorliegen. Mit einer optischen Zielerkennungstechnik können einzelne Zellen gezielt anvisiert und übertragen werden, ohne dabei die Zellvitalität entscheidend zu beeinflussen. Ein Vorteil des LIFT-Verfahrens ist die Integrierbarkeit weiterer Verfahren, zum Beispiel der Lichtmikroskopie und der Raman-Spektroskopie, mit denen Zellen weitergehend automatisiert untersucht werden können.

In einer bevorzugten Ausführungsform beträgt die Laserimpulsenergie beim LIFT-Verfahren 1 µJ bis 20 µJ, bevorzugt 1 µJ bis 15 µJ, bevorzugt 1 µJ bis 10 µJ, bevorzugt 2 µJ bis 10 µJ, bevorzugt 3 µJ bis 10 µJ, bevorzugt 5 µJ bis 10 µJ, bevorzugt 6 µJ bis 10 µJ, bevorzugt 8 µJ bis 10 µJ.

In einer weiteren bevorzugten Ausführungsform beträgt der Durchmesser des Laserbrennflecks beim LIFT-Verfahren 10 µm bis 200 µm, bevorzugt 15 µm bis 150 µm, bevorzugt 20 µm bis 100 µm, bevorzugt 25 µm bis 90 µm, bevorzugt 30 µm bis 80 µm, bevorzugt 40 µm bis 80 µm, bevorzugt 50 µm bis 70 µm.

Bevorzugt besteht die Trägerschicht des beim LIFT-Verfahren verwendeten Transferträgers aus Glas, bevorzugt aus Quarz, oder aus Kunststoff.

Bevorzugt besteht die Absorberschicht des beim LIFT-Verfahren verwendeten Transferträgers aus Titan, Gold oder einem anderen die Laserwellenlänge absorbierenden Stoff. Bevorzugt beträgt die Dicke der Absorberschicht des beim LIFT-Verfahren verwendeten Transferträgers 5 nm bis 300 nm, bevorzugt 5 nm bis 250 nm, bevorzugt 5 nm bis 200 nm, bevorzugt 5 nm bis 150 nm, bevorzugt 5 nm bis 120 nm, bevorzugt 5 nm bis 100 nm, bevorzugt 5 nm bis 80 nm, bevorzugt 5 nm bis 60 nm, bevorzugt 10 nm bis 50 nm, bevorzugt 10 nm bis 40 nm, bevorzugt 10 nm bis 30 nm, bevorzugt 10 nm bis 20 nm.

Gemäß der vorliegenden Erfindung weist der Transferträger keine Absorberschicht auf. Erfindungsgemäß dient die Transfermaterial-Schicht als Absorberschicht. Erfindungsgemäß werden bei der Verwendung der Transfermaterial-Schicht als Absorberschicht IR Laserstrahlquellen mit > 3 µm Wellenlänge verwendet.

Bevorzugt beträgt die Distanz zwischen dem Transferträger und dem Empfängerträger beim LIFT-Verfahren 20 µm bis 2000 µm, bevorzugt 25 µm bis 1800 µm, bevorzugt 30 µm bis 1600 µm, bevorzugt 40 µm bis 1400 µm, bevorzugt 50 µm bis 1200 µm, bevorzugt 60 µm bis 1100 µm, bevorzugt 70 µm bis 1100 µm, bevorzugt 80 µm bis 1000 µm, bevorzugt 90 µm bis 1000 µm, bevorzugt 100 µm bis 1000 µm, bevorzugt 200 µm bis 900 µm, bevorzugt 300 µm bis 800 µm, bevorzugt 400 µm bis 800 µm.

Erfindungsgemäß erfolgt in Schritt e) das Kultivieren der mindestens einen in Schritt c) selektierten Zelle in einem Expansionsmedium. Bevorzugt wird die mindestens eine in Schritt c) selektierte Zelle in Schritt e) mindestens eine Stunde, bevorzugt mindestens 2 Stunden, bevorzugt mindestens 4 Stunden, bevorzugt mindestens 6 Stunden, bevorzugt mindestens 8 Stunden, bevorzugt mindestens 10 Stunden, bevorzugt mindestens 12 Stunden, bevorzugt mindestens 24 Stunden, bevorzugt mindestens 2 Tage, bevorzugt mindestens 3 Tage, bevorzugt mindestens 4 Tage, bevorzugt mindestens 5 Tage, bevorzugt mindestens 6 Tage, bevorzugt mindestens 7 Tage, in einem Expansionsmedium kultiviert.

In einer bevorzugten Ausführungsform der vorliegenden Erfindung erfolgt in Schritt e) das Kultivieren der mindestens einen in Schritt c) selektierten Zelle in einem Protein- und Serumfreien Expansionsmedium. In einer bevorzugten Ausführungsform der vorliegenden Erfindung erfolgt in Schritt e) das Kultivieren der mindestens einen in Schritt c) selektierten Zelle in einem Protein- und Serumhaltigen Expansionsmedium.

Erfindungsgemäß erfolgt in Schritt f) das Charakterisieren der quantitativen Proteinexpression der in Schritt e) kultivierten mindestens einen Zelle auf Einzelzellebene mittels SERS und/oder zeitlich aufgelöster Raman-Spektroskopie.

In einer bevorzugten Ausführungsform der vorliegenden Erfindung erfolgt das Charakterisieren der quantitativen Proteinexpression der in Schritt e) kultivierten mindestens einen Zelle auf Einzelzellebene in Schritt f) im Expansionsmedium.

Erfindungsgemäß erfolgt in Schritt g) das Selektieren einer, bevorzugt mindestens einer, die gewünschte Zielprotein-Expression aufweisenden Zelllinie. Bevorzugt wird in Schritt g) genau eine die gewünschte Zielprotein-Expression aufweisenden Zelllinie selektiert. Bevorzugt werden in Schritt g) zwei, bevorzugt drei, bevorzugt vier, bevorzugt fünf, bevorzugt sechs, die gewünschte Zielprotein-Expression aufweisende Zelllinien selektiert. Besonders bevorzugt werden in Schritt g) beliebig viele die gewünschte Zielprotein-Expression aufweisende Zelllinien selektiert.

Die vorliegende Erfindung betrifft auch eine Vorrichtung zur Selektion von Zelllinien, umfassend
i) mindestens ein optisches System zur Raman-Spektroskopie auf Einzelzellebene,
ii) mindestens ein Laser Induced Forward Transfer (LIFT)-System zum Transfer von selektierten Zellen,
   wobei die Laserstrahlquelle des mindestens einen Laser Induced Forward Transfer (LIFT)-Systems eine Wellenlänge von > 3 µm aufweist, und wobei der in dem mindestens einen Laser Induced Forward Transfer (LIFT)-System verwendete Transferträger keine Absorberschicht aufweist und wobei die Transfermaterial-Schicht als Absorberschicht dient.

In einer bevorzugten Ausführungsform ist das mindestens eine optische System gemäß i) ein System zur Surface-Enhanced Raman-Spektroskopie auf Einzelzellebene.

Bevorzugt gelten die im Zusammenhang mit dem erfindungsgemäßen oder erfindungsgemäß bevorzugten Verfahren getroffenen Aussagen und aufgeführten Ausführungsformen mutatis mutandis auch für die Vorrichtung zur Selektion von eine gewünschte Zielprotein-Expression aufweisenden Zelllinien.

Weitere vorteilhafte Ausgestaltungen der Erfindung ergeben sich aus den Unteransprüchen.

Die vorliegende Erfindung wird im Folgenden anhand der Figuren näher erläutert.

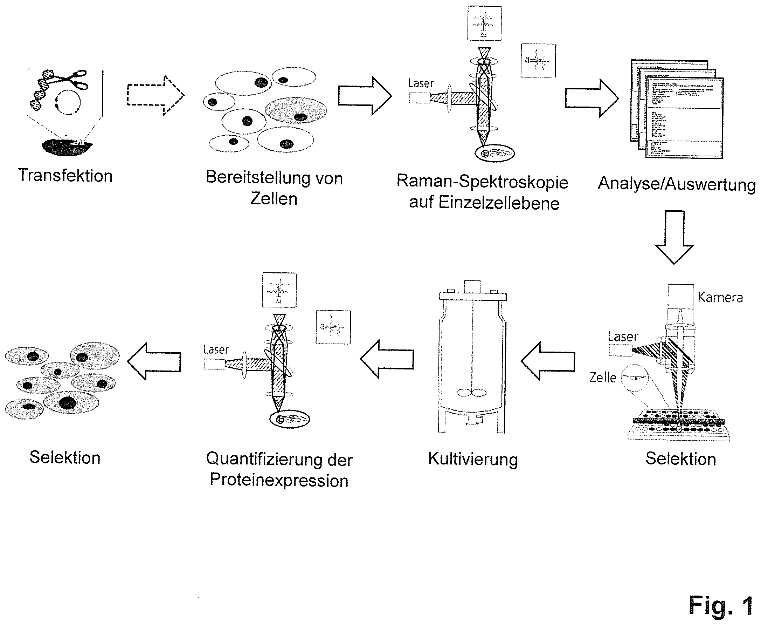

Figur 1 zeigt schematisch die Verfahrensschritte des erfindungsgemäßen Verfahrens zur Bereitstellung einer eine gewünschte Zielprotein-Expression aufweisenden Zelllinie. Dabei werden in einem ersten Schritt Zellen bereitgestellt (Bereitstellung von Zellen), die mindestens eine Nukleotidsequenz aufweisen, die mindestens ein Zielprotein kodiert. In einer bevorzugten Ausführungsform der vorliegenden Erfindung können die bereitgestellten Zellen in einem vorgeschalteten Verfahrensschritt durch Transfektion (Transfektion) von Zellen mit mindestens einer Nukleotidsequenz, die mindestens ein Zielprotein kodiert, erhalten werden. Die Proteinexpression der bereitgestellten Zellen wird anschließend auf Einzelzellebene mittels Raman-Spektroskopie charakterisiert (Raman-Spektroskopie auf Einzelzellebene). Daraufhin werden die erhaltenen Raman-Spektren analysiert (Analyse/Auswertung), mindestens eine eine gewünschte Zielprotein-Expression aufweisende Zelle selektiert (Selektion) und in ein Expansionsmedium transferiert. Die mindestens eine selektierte Zelle wird anschließend in einem Expansionsmedium kultiviert (Kultivierung). In einem weiteren Schritt erfolgt eine Charakterisierung der quantitativen Proteinexpression (Quantifizierung der Proteinexpression) der kultivierten Zellen auf Einzelzellebene mittels zeitaufgelöster Raman-Spektroskopie und die Selektion (Selektion) mindestens einer Zelllinie, die die gewünschte Zielprotein-Expression aufweist.

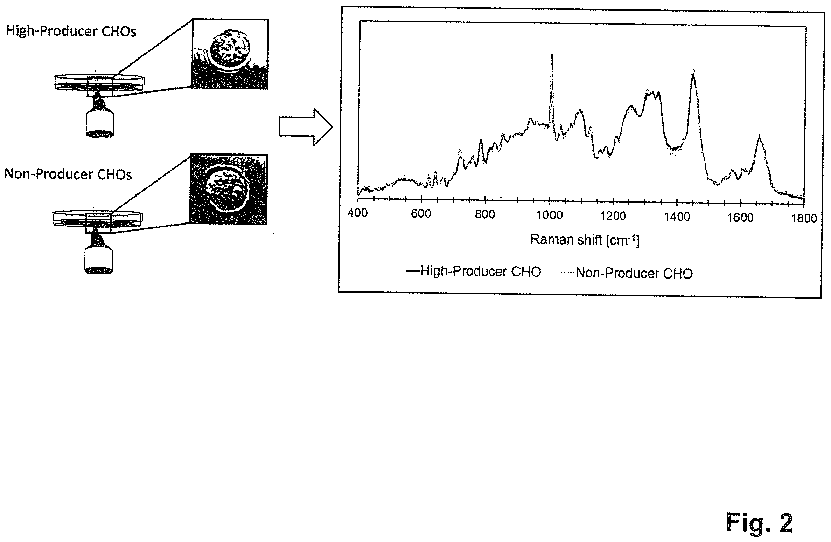

Figur 2 zeigt mittels Einzelzell-Raman-Spektroskopie erhaltene unterschiedliche Spektren von CHO-Zellen mit unterschiedlicher Zielprotein-Expression, nämlich einerseits High-Producer-CHO-Zellen (hohe Expression des Zielproteins) und andererseits Non-Producer-CHO-Zellen (keine Expression des Zielproteins).

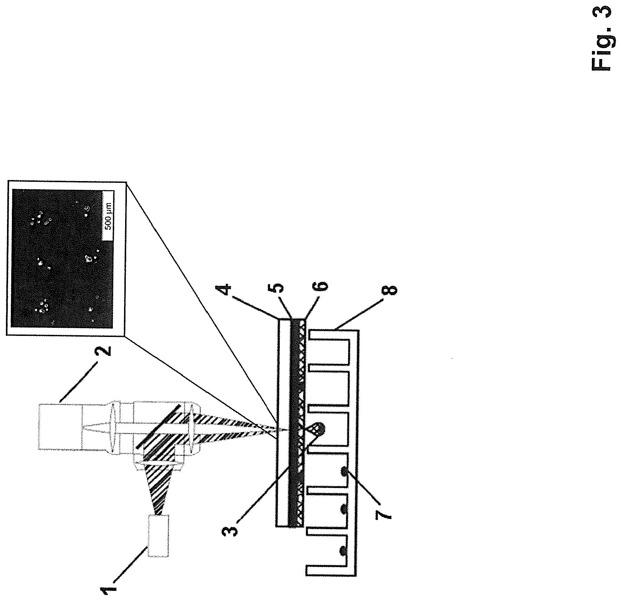

Figur 3 zeigt das zum Transfer von selektierten Einzelzellen verwendete LIFT-Verfahren. Dabei wird mittels eines Lasers (1) ein Laserimpuls bewirkt, der die Trägerschicht (4) eines Transferträger (4, 5, 6) passiert und zu einer kurzzeitigen lokalen Erwärmung der Absorberschicht (5) führt (nicht erfindungsgemäß), wodurch eine Dehnungswelle oder Dampfblase in der Transferschicht (6) erzeugt wird, die es ermöglicht, Zellen (3) gezielt über eine kurze Distanz auf einen Empfängerträger (8) zu übertragen und auf diese Weise selektierte Zellen (7) zu vereinzeln. Mittels einer optischen Zielerkennungstechnik (2) können mit dem Verfahren ganz gezielt Einzelzellen (3) anvisiert und einzeln übertragen werden.

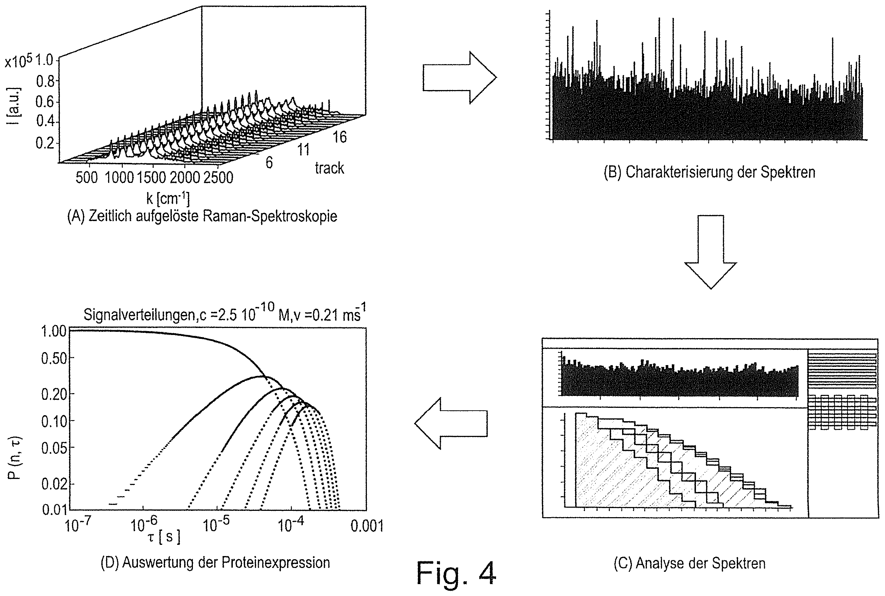

Figur 4 zeigt schematisch ein Verfahren zur Charakterisierung der quantitativen Proteinexpression auf Einzelzellebene. Dazu wird zunächst eine große Anzahl an Raman-Spektren einer einzelnen Probe mit geringen Belichtungszeiten in Form einer Zeitreihe aufgenommen ((A), Zeitlich aufgelöste Raman-Spektroskopie). In den erhaltenen Spektren werden anschließend relevante Merkmale bestimmt und für die jeweilige Pixelposition eine Zeitreihe generiert ((B), Charakterisierung der Spektren). In einem weiteren Schritt werden die Zeitreihen unter Variation der Bin-Breiten histogrammisiert ((C), Analyse der Spektren) und die so gewonnen Daten schließlich als Funktion der Zeit geplottet, um probenspezifische Informationen über die Zielprotein-Expression zu erhalten ((D), Auswertung der Proteinexpression).

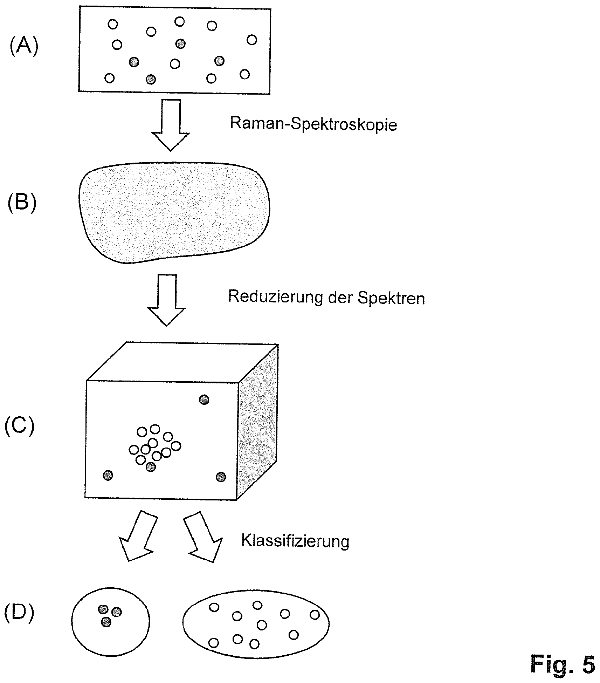

Figur 5 zeigt ein Verfahren zur Klassifizierung der in dem erfindungsgemäßen Verfahren mittels Raman-Spektroskopie charakterisierten Zellen. Dabei werden auf einem Träger befindliche Einzelzellen zunächst mittels Raman-Spektroskopie charakterisiert ((A), Raman-Spektroskopie). Daraufhin werden die Feature-Space-Dimensionen der erhaltenen Raman-Spektren reduziert ((B), Reduzierung der Spektren) und mittels Ensemble Machine Learning Methoden klassifiziert ((C), Klassifizierung), sodass schließlich ausgehend von den aufgenommenen Raman-Spektren eine Einteilung der charakterisierten Zellen in Abhängigkeit von der Zielprotein-Expression möglich ist (D).

## Patentansprüche

1. Verfahren zur Bereitstellung einer eine gewünschte Zielprotein-Expression aufweisenden Zelllinie, wobei das Verfahren folgende Schritte umfasst:
a) Bereitstellen von Zellen, die jeweils mindestens eine Nukleotidsequenz aufweisen, die mindestens ein Zielprotein kodiert,
b) Charakterisieren der Proteinexpression der in Schritt a) bereitgestellten Zellen auf Einzelzellebene mittels Raman-Spektroskopie,
c) Selektieren mindestens einer Zelle, die eine gewünschte Zielprotein-Expression aufweist,
d) Transferieren der mindestens einen in Schritt c) selektierten Zelle in ein Expansionsmedium mittels Laser Induced Forward Transfer (LIFT), wobei der verwendete Transferträger keine Absorberschicht aufweist, wobei die Transfermaterial-Schicht als Absorberschicht dient und wobei für den Laser Induced Forward Transfer (LIFT) eine Laserstrahlquelle mit einer Wellenlänge von > 3 µm verwendet wird,
e) Kultivieren der mindestens einen selektierten Zelle in einem Expansionsmedium,
f) Charakterisieren der quantitativen Proteinexpression der in Schritt e) kultivierten mindestens einen Zelle auf Einzelzellebene mittels zeitaufgelöster Raman-Spektroskopie, und
g) Selektieren einer die gewünschte Zielprotein-Expression aufweisenden Zelllinie.

2. Verfahren nach Anspruch 1, wobei das Verfahren markerfrei ist.

3. Verfahren nach Anspruch 1 oder 2, wobei vor Schritt a) eine Transfektion von Zellen mit mindestens einer Nukleotidsequenz, die mindestens ein Zielprotein kodiert, erfolgt.

4. Verfahren nach einem der vorherigen Ansprüche, wobei die Charakterisierung der Proteinexpression der Zellen in Schritt b) mittels Surface-Enhanced Raman-Spektroskopie erfolgt.

5. Verfahren nach einem der vorherigen Ansprüche, wobei nach Schritt b) eine Klassifizierung der in Schritt b) charakterisierten Zellen mittels Hauptkomponentenanalyse (PCA) erfolgt.

6. Verfahren nach einem der vorherigen Ansprüche, wobei nach Schritt b) eine Klassifizierung der in Schritt b) charakterisierten Zellen mittels Ensemble Machine Learning Methoden und Analyse mittels neuronaler Netzwerke erfolgt.

7. Verfahren nach Anspruch 6, wobei die Ensemble Machine Learning Methoden unüberwacht oder teilüberwacht sind.

8. Verfahren nach einem der vorherigen Ansprüche, wobei das Charakterisieren der quantitativen Proteinexpression in Schritt f) im Expansionsmedium erfolgt.

9. Vorrichtung zur Selektion von eine gewünschte Zielprotein-Expression aufweisenden Zelllinien, umfassend
i) mindestens ein optisches System zur Raman-Spektroskopie auf Einzelzellebene,
ii) mindestens ein Laser Induced Forward Transfer (LIFT)-System zum Transfer von selektierten Zellen,
wobei die Laserstrahlquelle des mindestens einen Laser Induced Forward Transfer (LIFT)-Systems eine Wellenlänge von > 3 µm aufweist, und wobei der in dem mindestens einen Laser Induced Forward Transfer (LIFT)-System verwendete Transferträger keine Absorberschicht aufweist und wobei die Transfermaterial-Schicht als Absorberschicht dient.

10. Vorrichtung nach Anspruch 9, wobei das mindestens eine optische System ein System zur Surface-Enhanced Raman-Spektroskopie auf Einzelzellebene ist.

## Claims

1. A method of providing a cell line having a desired target protein expression, the method comprising the steps of:
a) providing cells each having at least one nucleotide sequence encoding at least one target protein,
b) characterising the protein expression of the cells provided in step a) at the single cell level by Raman spectroscopy,
c) selecting at least one cell having a desired target protein expression,
d) transferring the at least one cell selected in step c) into an expansion medium by means of Laser Induced Forward Transfer (LIFT), wherein the transfer carrier used does not have an absorber layer, wherein the transfer material layer serves as an absorber layer and wherein a laser source with a wavelength of > 3 µm is used for the Laser Induced Forward Transfer (LIFT),
e) cultivating the at least one selected cell in an expansion medium,
f) characterising the quantitative protein expression of the at least one cell cultivated in step e) on a single cell plane by means of time-resolved Raman spectroscopy, and
g) selecting a cell line having the desired target protein expression.

2. The method according to claim 1, wherein the method is marker-free.

3. The method according to claim 1 or 2, wherein prior to step a) a transfection of cells with at least one nucleotide sequence takes place, which encodes at least one target protein.

4. The method according to one of the preceding claims, wherein the characterisation of the protein expression of the cells in step b) takes place by means of surface-enhanced Raman spectroscopy.

5. The method according to one of the preceding claims, wherein after step b), a classification of the cells **characterised in** step b) takes place by means of principal component analysis (PCA).

6. The method according to one of the preceding claims, wherein after step b) a classification of the cells **characterised in** step b) takes place by means of ensemble machine learning methods and analysis by means of neural networks.

7. The method according to claim 6, wherein the ensemble machine learning methods are unsupervised or semi-supervised.

8. The method according to one of the preceding claims, wherein the characterisation of the quantitative protein expression in step f) takes place in the expansion medium.

9. A system for selecting cell lines having a desired target protein expression, comprising
i) at least one optical system for Raman spectroscopy at the single cell level,
ii) at least one Laser Induced Forward Transfer (LIFT) system for the transfer of selected cells,
wherein the laser source of the at least one Laser Induced Forward Transfer (LIFT) system has a wavelength of > 3 µm, and wherein the transfer carrier used in the at least one Laser Induced Forward Transfer (LIFT) system does not have an absorber layer and wherein the transfer material layer serves as an absorber layer.

10. The system according to claim 9, wherein the at least one optical system is a system for surface-enhanced Raman spectroscopy at the single cell level.

## Revendications

1. Un procédé pour fournir une lignée cellulaire ayant une expression d'une protéine cible souhaitée, le procédé comprenant les étapes suivantes :
a) fournir des cellules ayant chacune au moins une séquence nucléotidique codant pour au moins une protéine cible,
b) caractériser l'expression d'une protéine des cellules fournies à l'étape a) au niveau d'une seule cellule par spectroscopie Raman,
c) choisir la au moins cellule ayant l'expression d'une protéine cible souhaitée,
d) transférer au moins la cellule choisie à l'étape c) dans un milieu d'expansion au moyen d'un Laser Induced Forward Transfer (LIFT), dans lequel le support de transfert utilisé n'a pas de couche absorbante, dans lequel la couche de matériau de transfert sert de couche absorbante et dans lequel une source laser d'une longueur d'onde > 3 µm est utilisée pour le Laser Induced Forward Transfer (LIFT),
e) cultiver la au moins cellule choisie dans un milieu d'expansion,
f) caractériser l'expression d'une protéine quantitative de l'au moins cellule cultivée à l'étape e) sur un plan cellulaire unique au moyen d'une spectroscopie Raman résolue dans le temps, et
g) choisir une lignée cellulaire ayant l'expression d'une protéine cible souhaitée.

2. Le procédé selon la revendication 1, dans lequel le procédé est exempt de marqueurs.

3. Le procédé selon la revendication 1 ou 2, dans lequel avant l'étape a) a lieu une transfection des cellules avec au moins une séquence nucléotidique qui code pour au moins une protéine cible.

4. Le procédé selon l'une quelconque des revendications précédentes, dans lequel la caractérisation de l'expression d'une protéine des cellules à l'étape b) a lieu au moyen de la spectroscopie Raman améliorée par la surface.

5. Le procédé selon l'une quelconque des revendications précédentes, dans lequel après l'étape b), une classification des cellules caractérisées à l'étape b) a lieu au moyen d'une analyse en composantes principales (ACP).

6. Le procédé selon l'une quelconque des revendications précédentes, dans lequel après l'étape b), une classification des cellules caractérisées à l'étape b) a lieu au moyen de méthodes d'apprentissage machine d'ensemble et d'analyse au moyen de réseaux neuronaux.

7. Le procédé selon la revendication 6, dans lequel les méthodes d'apprentissage machine d'ensemble sont non supervisées ou semi-supervisées.

8. Le procédé selon l'une quelconque des revendications précédentes, dans lequel la caractérisation de l'expression quantitative d'une protéine à l'étape f) a lieu dans le milieu d'expansion.

9. Un dispositif pour choisir des lignées cellulaires ayant une expression d'une protéine cible souhaitée, comprenant
i) au moins un système optique pour la spectroscopie Raman au niveau de la cellule unique,
ii) au moins un système de Laser Induced Forward Transfer (LIFT) pour le transfert des cellules choisies,
dans lequel la source laser du au moins un système de Laser Induced Forward Transfer (LIFT) a une longueur d'onde > 3 µm, et dans lequel le support de transfert utilisé dans le au moins système de Laser Induced Forward Transfer (LIFT) n'a pas de couche absorbante et dans lequel la couche de matériau de transfert sert de couche absorbante.

10. Le dispositif selon la revendication 9, dans lequel le au moins système optique est un système de spectroscopie Raman améliorée par la surface au niveau de la cellule unique.
